(19) **Europäisches Patentamt**

**European Patent Office**

**Office européen des brevets**

(11) Publication number: **0 378 887 B1**

# EUROPEAN PATENT SPECIFICATION

(45) Date of publication of patent specification: **13.09.95**  (51) Int. Cl.⁶: **C10G 45/64**, B01J 29/44

(21) Application number: **89300410.1**

(22) Date of filing: **17.01.89**

(54) **A process for paraffin isomerization of a distillate range hydrocarbon feedstock.**

(43) Date of publication of application:
**25.07.90 Bulletin 90/30**

(45) Publication of the grant of the patent:
**13.09.95 Bulletin 95/37**

(84) Designated Contracting States:
**AT BE DE FR GB IT NL SE**

(56) References cited:
EP-A- 0 016 554      EP-A- 0 102 716
EP-A- 0 161 833      FR-A- 2 313 980
US-A- 4 016 245      US-A- 4 372 839
US-A- 4 556 477      US-A- 4 814 543

(73) Proprietor: **MOBIL OIL CORPORATION**
**150 East 42nd Street**
**New York**
**New York 10017 (US)**

(72) Inventor: **Chen, Nai Yuen**
**4 Forrest Central Drive**
**R.D. Titusville**
**New Jersey 08560 (US)**
Inventor: **Garwood, William Everett**
**125 Warwick Road**
**Haddonfield**
**New Jersey 08033 (US)**
Inventor: **McCullen, Sharon Brawner**
**119 Colonial Drive**
**Newton**
**Pennsylvania 18940 (US)**

(74) Representative: **Colmer, Stephen Gary**
**Mobil Services Company Limited**
**Patent Department**
**Mobil House**
**54-60 Victoria Street**
**London SW1E 6OB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to a process for paraffin isomerization, for example hydroisomerisation of a distillate range hydrocarbon feedstock.

Various catalytic processes have been proposed for isomerizing n-paraffins so as to lower the pour point of distillate range hydrocarbon feedstocks. However, many available feedstocks contain nitrogen impurities (typically at least 20 ppm) which tend to poison conventional paraffin isomerization catalysts. An object of the present invention is therefore to obviate or alleviate this problem.

Accordingly, the present invention resides in a process for paraffin isomerization of a hydrocarbon feedstock having a boiling range of 165 - 343 °C and a paraffinic content of molecules with not more than 20 carbon atoms, and preferably no more than 16 carbon atoms, which includes contacting the paraffin-containing feedstock with a crystalline aluminosilicate zeolite catalyst having pore openings defined by: (1) a ratio of sorption of n-hexane to o-xylene, on a volume percent basis, of greater than 3, which sorption is determined at a $P/P_o$ of 0.1 and at a temperature of 50 °C for n-hexane and 80 °C for o-xylene and (2) by the ability of selectively cracking 3-methylpentane (3MP) in preference to the doubly branched 2,3-dimethylbutane (DMB) at 538 °C (1000 °F) and 101 kPa (1 atmosphere) pressure from a 1/1/1 weight ratio mixture of n-hexane/3-methyl-pentane/2,3-dimethylbutane, with the ratio of rate constants $k_{3MP}/k_{DMB}$ determined at a temperature of 538 °C (1000 °F) being in excess of 2, said catalyst including 0.01 to 10% wt. of a platinum Group metal incorporated into said zeolite by ion exchange, and having a zeolite $SiO_2/Al_2O_3$ ratio of at least 20:1, and said contacting being effected at a temperature of 199 - 454 °C (390 to 850 °F) and a pressure of 790 - 7000 kPa (100 to 1,000 psig).

Preferably, said contacting is effected at a pressure of 1825 - 4240 kPa (250 - 600 psig).

The expression, "$P/P_o$, as utilized herein, is accorded its usual significance as described in the literature, for example, in "The Dynamical Character of Adsorption" by J.H. deBoer, 2nd Edition, Oxford University Press (1968) and is the relative pressure defined as the ratio of the partial pressure of sorbate to the vapor pressure of sorbate at the temperature of sorption. The ratio of the rate constants, $k_{3MP}/k_{DMB}$, is determined from 1st order kinetics, in the usual manner, by the following equation:

$$k = (1/T_c)\ln(1/1\text{-}\epsilon)$$

where k is the rate constant for each component, $T_c$ is the constant time and $\epsilon$ is the fractional conversion of each component.

The zeolite catalysts described above are effective for the isomerization of n-paraffins and are resistant to nitrogen poisons, especially those found in distillate range feedstocks, as a result of their specific pore size openings which are effective to exclude most nitrogen containing organic molecules from entering therein. Inasmuch as medium pore zeolites have in the past been considered as somewhat limited for purposes of isomerization as a result of their pore size, the present invention provides an unexpectedly efficient procedure and catalyst for effectively isomerizing paraffinic feedstocks, especially in hydrocarbon distillate range feedstock having nitrogen poisons.

The group of medium pore zeolites which can be used in the process of the present invention includes ZSM-22, ZSM-23, and ZSM-35. ZSM-22 is described in U.S. Patent No. 4556477, ZSM-23 is described in U.S. Patent No. 4076842 and ZSM-35 is described in U.S. Patent No. 4016245.

The metals used in the catalyst of the present invention are selected from platinum, palladium, iridium, osmium, rhodium and rhuthenium, and are preferably present in the amount of from 0.1% to 3% by weight of the zeolite. The zeolite can be supported in a binder selected from one of silica, alumina, silica-alumina, and titania.

When the feedstock is distillate range feedstock, having a boiling point of 165 - 343 °C (330 to 650 °F), the catalyst of the present invention has been shown to resist poisoning by nitrogenous compounds under the conditions necessary to isomerize n-paraffins in the feedstock.

Furthermore, a hydrocarbon feedstock having a substantial paraffinic content can be converted to reduce the boiling point 5-11 °C (10 to 20 °F) without the unwanted side effects of significant cracking which normally results from high temperature, high pressure procedures in the presence of a zeolitic catalyst. As a result, the feedstock can be treated without significant reduction of distillate range yield. The product resulting from this process contains a significantly greater amount of isoparaffinic compounds, i.e., branched hydrocarbon chains, than straight hydrocarbon chains. This is unlike the product resulting from hydrocracking processes which includes a greater amount of straight chain molecules. Consequently, the yield of nonparaffinic distillate is significantly increased.

Thus, while other procedures have been found to be useful in reducing the paraffinic content by hydrocracking, the present invention has been found to be unexpectedly efficient in reducing the n-paraffin content by hydroisomerization while maintaining high distillate range yields.

The following Examples illustrate the invention.

### Example 1 (Comparative)

The isomerization selectivity of Pt/ZSM-5/Al$_2$O$_3$ was measured using N-hexadecane at 3550 kPa (550 psig) 277°C (531°F), H$_2$/HC = 13.1 and 1 liquid hourly space velocity (LHSV). At 77% n-C$_{16}$ conversion, the selectivity (the ratio of the i-C$_{16}$ yield and the n-C$_{16}$ conversion) was only 0.30. Benzoquinoline was then added to the n-C$_{16}$ feed in an amount to obtain 20 ppm nitrogen. It was necessary to increase the reactor temperature 30°C (54°F) to maintain the n-C$_{16}$ conversion at 77 wt.%. The isomerization selectivity increased to 0.43.

### Example 2

The isomerization selectivity of Pt/ZSM-22/Al$_2$O$_3$ was measured as described in Example 1. 85 wt.% conversion of n-C$_{16}$ was obtained at 302°C (576°F) and the isomerization selectivity was 0.76. When 20 ppm nitrogen was added as benzoquinoline, the reactor temperature was increased 29°C (52°F) to maintain conversion, no change in isomerization selectivity was observed. Thus this catalyst showed better isomerization selectivity compared to ZSM-5 for a clean feed and in the presence of a nitrogen poison.

### Example 3

The performance of Pt/ZSM-23/Al$_2$O$_3$ for isomerization was tested as described in Example 1. At 320°C (608°F), 69 wt.% conversion of n-C$_{16}$ was observed with 0.60 isomerization selectivity. The reactor temperature was increased only 20°C (36°F) to maintain the n-C$_{16}$ conversion after benzoquinoline was added. This catalyst shows higher isomerization selectivity and better nitrogen resistance compared to Pt/ZSM-5/Al$_2$O$_3$ in Example 1.

### Example 4

Pt/ZSM-35/Al$_2$O$_3$ was used to isomerize n-C$_{16}$ at the conditions described in Example 1. 76 wt.% n-C$_{16}$ conversion was observed at 307°C (585°F) with 0.50 isomerization selectivity. When 20 ppm nitrogen as benzoquinoline was added the reactor temperature was increased by 60°C (108°F) to maintain conversion, however, the isomerization selectivity increased to 0.70. Although a larger temperature increase was required to maintain n-C$_{16}$ conversion activity compared to Pt/ZSM-5/Al$_2$O$_3$, the isomerization selectivity was much higher for Pt/ZSM-35/Al$_2$O$_3$ for a clean feed and for a feed containing nitrogen.

The results of the above Examples are summarized in Table 1 below.

TABLE I

|  | Example 1 | Example 2 | Example 3 | Example 4 |
|---|---|---|---|---|
| n-C$_{16}$ Conversion w/o Nitrogen | 77% | 85% | 69% | 76% |
| n-C$_{16}$ Conversion w/ Nitrogen | 77% | 85% | 69% | 76% |
| i-C$_{16}$* Selectivity w/o Nitrogen | 0.30 | 0.76 | 0.60 | 0.50 |
| i-C$_{16}$* Selectivity w/ Nitrogen | 0.43 | 0.76 | 0.60 | 0.70 |

*Isomerization selectivity is defined as the ratio of the i-C$_{16}$ yield to the n-C$_{16}$ conversion.

## Claims

1. A process for paraffin isomerization of a distillate range hydrocarbon feedstock having a boiling range of 165 - 343°C (330 - 650°F) and a paraffinic content of molecules with not more than 20 carbon atoms and containing nitrogen impurities of at least 20 ppm comprising:

contacting said paraffin-containing feedstock with a catalyst including a crystalline zeolite catalyst having pore openings defined by: (1) a ratio of sorption of n-hexane to o-xylene, on a volume percent basis, of greater than 3, which sorption is determined at a $P/P_o$ of 0.1 and at a temperature of 50°C for n-hexane and 80°C for o-xylene and (2) by the ability of selectively cracking 3-methylpentane (3MP) in preference to the 2,3dimethylbutane (DMB) at 538°C (1000°F) and 101 kPa (1 atmosphere) pressure from a 1/1/1 weight ratio mixture of n-hexane/3-methyl-pentane/2,3dimethylbutane (DMB), with the ratio rate constants $k_{3MP}/k_{DMB}$ determined at a temperature of 538°C (1000°F) being in excess of 2, said catalyst including 0.01 to 10% wt. of a platinum Group metal incorporated into said zeolite by ion exchange, and having a zeolite $SiO_2/Al_2O_3$ ratio of at least 20:1, and said contacting being effected at a temperature of 199 - 454°C (390 to 850°F) and a pressure of 790 - 7000 kPa (100 to 1,000 psig).

2. The process of Claim 1 wherein said crystalline aluminosilicate catalyst is selected from ZSM-22, ZSM-23 and ZSM-35.

3. The process of Claim 1 or Claim 2 wherein said metal is selected from Pt, Pd, Ir, Os, Rh and Ru.

4. The process of any preceding Claim wherein said metal content is from 0.1% to 3.0% by weight.

5. The process of any preceding Claim wherein said zeolite is supported in a binder selected from silica, alumina, silica-alumina, and titania.

6. The process of any preceding Claim wherein said paraffin content has molecules of no greater than 16 carbon atoms.

7. A process according to claim 1 which is a hydroisomerisation process and wherin the catalyst comprises ZSM-22, ZSM-23 or ZSM-25.

**Patentansprüche**

1. Verfahren zur Paraffinisomerisierung eines Kohlenwasserstoffbeschickungsmaterials im Destillatbereich mit einem Siedebereich von 165 bis 343°C (330 bis 650°F) und einem Paraffingehalt von Molekülen mit nicht mehr als 20 Kohlenstoffatomen, das Stickstoffverunreinigungen von mindestens 20 ppm enthält, welches umfaßt:
Kontakt des paraffinhaltigen Ausgangsmaterials mit einem Katalysator, der einen kristallinen Zeolithkatalysator mit Porenöffnungen umfaßt, die definiert werden durch: (1) ein Verhältnis der Sorption von n-Hexan zu o-xylol auf Volumenprozentbasis von mehr als 3, wobei die Sorption bei $P/P_o$ von 0,1 und einer Temperatur von 50°C für n-Hexan und 80°C für o-Xylol bestimmt wurde, und (2) die Fähigkeit des bevorzugten selektiven Crackens von 3-Methylpentan (3MP) gegenüber 2,3-Dimethylbutan (DMB) bei 538°C (1000°F) und einem Druck von 101 kPa (1 Atmosphäre) aus einer Mischung von n-Hexan/3-Methylpentan/2,3-Dimethylbutan (DMP) mit einem Gewichtsverhältnis von 1/1/1, wobei das Verhältnis der Geschwindigkeitskonstanten $k_{3MP}/k_{DMB}$, das bei einer Temperatur von 538°C (1000°F) bestimmt wurde, mehr als 2 beträgt, der Katalysator 0,01 bis 10 Gew.-% eines Metalls der Platingruppe enthält, das durch Ionenaustausch in den Zeolith eingeführt wurde, und ein $SiO_2/Al_2O_3$-Verhältnis des Zeoliths von mindestens 20:1 aufweist, und der Kontakt bei einer Temperatur von 199 bis 454°C (390 bis 850°F) und einem Druck von 790 bis 7000 kPa (100 bis 1000 psig) erfolgt.

2. Verfahren nach Anspruch 1, wobei der kristalline Aluminosilicatkatalysator aus ZSM-22, ZSM-23 und ZSM-35 ausgewählt ist.

3. Verfahren nach Anspruch 1 oder 2, wobei das Metall aus Pt, Pd, Ir, Os, Rh und Ru ausgewählt ist.

4. Verfahren nach einem der vorstehenden Ansprüche, wobei der Metallgehalt 0,1 bis 3,0 Gew.-% beträgt.

5. Verfahren nach einem der vorstehenden Ansprüche, wobei der Zeolith in einem Bindemittel getragen wird, das aus Siliciumdioxid, Aluminiumoxid, Siliciumdioxid-Aluminiumoxid und Titanoxid ausgewählt ist.

6. Verfahren nach einem der vorstehenden Ansprüche, wobei der Paraffingehalt Moleküle mit nicht mehr als 16 Kohlenstoffatomen aufweist.

**7.** Verfahren nach Anspruch 1, das ein Hydroisomerisierungsverfahren ist, und wobei der Katalysator ZSM-22, ZSM-23 oder ZSM-25 umfaßt.

## Revendications

**1.** Un procédé d'isomérisation de paraffines contenues dans une charge hydrocarbonée dans la gamme des distillats dont le point d'ébullition est compris entre 165 et 343°C (330 - 650°F) et contenant des paraffines dont la molécule ne renferme pas plus de 20 atomes de carbone et contenant au moins 20 ppm d'impuretés azotées, lequel procédé consiste à mettre la charge contenant des paraffines au contact d'un catalyseur zéolitique cristallin dont les ouvertures de pores sont définies par:

(1) un rapport sorption de n-hexane/sorption de o-xylène, exprimé en pourcentage volumétrique, supérieur à 3, cette sorption étant déterminée pour $P/P_0 = 0,1$ et à une température de 50°C pour le n-hexane et de 80°C pour le o-xylène; et

(2) l'aptitude au craquage sélectif du 3-méthylpentane (3MP), de préférence à celui du 2,3-diméthylbutane (DMB), à 538°C (1000°F) et à une pression de 101 kPa (1 atmosphère) à partir d'un mélange en proportions pondérales 1/1/1 n-hexane/3-méthylpentane/2,3-diméthylbutane, le rapport des constantes de vitesse $k_{3MP}/k_{DMB}$, déterminé à une température de 538°C (1000°F), étant supérieur à 2 et ledit catalyseur comprenant de 0,01 à 10% en poids d'un métal du groupe du platine incorporé dans ladite zéolite par échange d'ions, et présentant un rapport $SiO_2/Al_2O_3$ de la zéolite au moins égal à 20/1, et ledit contact étant effectué à une température de 199 à 454°C (390 à 850°F) et une pression de 790 à 7000 kPa (100 à 1000 psig).

**2.** Le procédé selon la revendication 1, dans lequel ledit catalyseur à base d'aluminosilicate cristallin est choisi parmi ZSM-22, ZSM-23 et ZSM-35.

**3.** Le procédé selon la revendication 1 ou la revendication 2, dans lequel ledit métal est choisi parmi Pt, Pd, Ir, Os, Rh et Ru.

**4.** Le procédé selon l'une quelconque des revendications précédentes, dans lequel ladite teneur en métal est comprise entre 0,1 et 3,0% en poids.

**5.** Le procédé selon l'une quelconque des revendications précédentes, dans lequel la zéolite est supportée par un liant choisi parmi silice, alumine, silice-alumine et titane.

**6.** Le procédé selon l'une quelconque des revendications précédentes, dans lequel les paraffines sont formées de molécules ne renfermant pas plus de 16 atomes de carbone.

**7.** Un procédé selon la revendication 1, qui est un procédé d'isomérisation et dans lequel le catalyseur comprend ZSM-22, ZSM-23 et ou ZSM-35.